Europäisches Patentamt

European Patent Office

Office européen des brevets

(19)

(11) **EP 0 719 567 B1**

(12) **EUROPÄISCHE PATENTSCHRIFT**

(45) Veröffentlichungstag und Bekanntmachung des Hinweises auf die Patenterteilung:
**07.03.2001   Patentblatt 2001/10**

(51) Int Cl.⁷: **A61N 1/365**

(21) Anmeldenummer: **95250314.2**

(22) Anmeldetag: **22.12.1995**

(54) **Herzschrittmacher mit einer Einrichtung zum Feststellen der körperlichen Belastung**

Cardiac pacemaker with a device for determining the patient's effort

Stimulateur cardiaque avec un système pour déterminer l'effort du patient

(84) Benannte Vertragsstaaten:
**DE ES FR GB IT**

(30) Priorität: **29.12.1994   DE 4447447**

(43) Veröffentlichungstag der Anmeldung:
**03.07.1996   Patentblatt 1996/27**

(73) Patentinhaber: **St. Jude Medical AB**
**175 84 Järfälla (SE)**

(72) Erfinder:
• **Stangl, Karl, Dr. med.**
  **D-10623 Berlin (DE)**

• **Laule, Michael, Dr. med.**
  **D-13465 Berlin (DE)**
• **Heinze, Roland, Dipl.-Ing.**
  **D-10555 Berlin (DE)**

(74) Vertreter: **Pfenning, Meinig & Partner**
**Kurfürstendamm 170**
**10707 Berlin (DE)**

(56) Entgegenhaltungen:
**EP-A- 0 215 731          EP-A- 0 452 733**
**WO-A-89/06990          WO-A-93/20889**
**US-A- 5 360 436**

**Beschreibung**

Die Erfindung betrifft einen Herzschrittmacher nach dem Oberbegriff des Hauptanspruchs.

Im Stand der Technik sind eine Vielzahl von Verfahren zur Anpassung der Stimulationsfrequenz von Herzschrittmachern an die Belastungssituation des Patienten bekannt. Dabei ist zu unterscheiden zwischen Systemen, die nichtphysiologische Parameter wie etwa die Bewegung (Aktivität) des Körpers, und solchen Systemen, die physiologische Parameter des Körpers wie etwa den Blutdruck oder die Bluttemperatur zur Frequenzregelung nutzen. Nichtphysiologisch frequenzadaptierende Systeme haben den Nachteil, daß sie zu unphysiologischen Stimulationsfrequenzen führen können (Treppauf-Treppab-Paradoxon). Physiologisch regelnde Sensoren dagegen haben den Nachteil, daß sie spezielle Sensoren erfordern, um geeignete Regelparameter des Herz-Kreislaufsystems messen zu können, und diese Sensoren nicht die erforderliche Langzeitstabilität besitzen. Deshalb gibt es eine Vielzahl von methodischen Ansätzen, auch unspezifische Meßverfahren, die mit langzeitstabilen Standardkathetern durchzuführen sind, wie die intrakardiale Impedanzmessung für die Schrittmacher- und Defibrillatorsteuerung nutzbar zu machen. Wesentliches Merkmal dieser Ansätze ist, daß sie spezielle Methoden anwenden, aus dem polymorphen Signal die Signalinhalte zu filtern, die wiederum eindeutig physiologisch definierbaren Vorgängen zuzuordnen sind. Dabei ist zu unterscheiden zwischen zwei Konzepten:

1. Passive Messung intrakardialer und intrathorakaler Meßwerte ohne gezielte Beeinflussung der Meßstrecke durch den Stimulator.

2. Aktive Messung intrakardialer Meßparameter mit definierter Beeinflussung kardiovasculärer Funktionen durch den Stimulator.

Da aktiv messende Systeme, etwa durch Modulation der Stimulationsfrequenz des Herzmuskels, den Vorteil haben, durch die phasensynchrone Analyse nichtkardiale Störgrößen zu unterdrücken, sind sie für die Nutzung physiologischer Funktionsparameter zur Frequenzregelung vorzuziehen.

Bei aktiv messenden Systemen ist es bekannt, frequenzwechselbezogene Signalauswertung von Aktionsparametern des Herzens zur Analyse intrakardialer Impedanzsignale für die Frequenzregelung in Herzschrittmachern zu nutzen. Hierbei wird vorgeschlagen, den Signalverlauf eines intrakardialen Meßparameters während eines Herzpulses (n+1) in Abhängigkeit von Frequenzänderungen $\Delta HR$ eines einzelnen vorhergehenden Pulses (n) zu bewerten. Ergänzend wird vorgeschlagen, auch die Differenz der Signaländerungen zweier aufeinanderfolgender Pulse (n+1) und (n+2) in Abhängigkeit von der Frequenz von Puls (n) zu verwenden. Zur Normierung der Einzel- oder Differenzwerte wird der Quotient aus dem jeweiligen Meßwert und einem bei hoher Frequenzänderung zu bestimmenden maximalen Änderungswert berechnet. Ein wesentlicher Nachteil dieser Anordnung ergibt sich aus der Tatsache, daß diese Methode nur bei Meßsignalen erfolgreich ist, bei denen eine exakter Trennung zwischen volumen- und druckspezifischen Änderungen möglich ist. Dies wird aber bei Impedanzmessung mit Hilfe eines unipolaren Katheters durch die Tatsache begrenzt, daß neben der Form und dem Volumen des Ventrikels vor allem Druckänderungen an den Elektroden den Signalverlauf beeinflussen. Um diese Einflüsse zu kompensieren, ist es notwendig, bei der Einstellung des Systems eine Trennung der druck- und volumenabhängigen Signalanteile durchzuführen. Die bekannte Anordnung gibt keinen Hinweis, wie die Trennung durchgeführt werden soll.

Weiterhin ist bekannt, mit periodischen Wechseln der Stimulationsfrequenz eine Optimierung der Frequenzregelung durchzuführen, indem die synchron zu den Frequenzwechseln auftretenden Änderungen des Herzzeitvolumens oder dazu proportionale Meßwerte ausgewertet werden. Diese bekannte Vorgehensweise hat den Nachteil, daß die Zeitkonstante der Messung des Herzzeitvolumens mit der geforderten Genauigkeit derart steigt, daß die Optimierung nur in langen Phasen gleichmäßiger Belastung möglich ist, also keine kurzfristigen Adaptierungen an die Belastung durchführbar ist (WO 89/06990).

EP 0 215 731 A2 beschreibt einen Herzschrittmacher bei dem Messwertaufnehmer und nachfolgende Verarbeitungswege an die individuellen Gegebenheiten angepasst und insbesondere auch bei Änderungen der Betriebsbedingungen anpaßbar sind. Dabei ist eine Telemetrie-Schnittstelle vorgesehen, wodurch externe Messwege z.B. zur Eichung verwendet werden können. Es kann direkt die körperliche Belastung erfasst werden, die in Zuordnung zu den physiologischen Messgrößen bevorzugt in tabellarischer Form abgespeichert wird, wobei die gespeicherten Werte später zur Einstellung der Frequenz des Schrittmachers verwendet werden.

In der WO 93/20889 ist ein Herzschrittmacher offenbart, der die Herzimpedanz erfaßt, wobei ein Prozessor die Impedanzsignalform abtastet und einen Bitstrom erzeugt, dessen Anzahl an Bits einer Änderung der Impedanzsignalform entspricht. Impedanzänderungen werden auf Atemänderungen hinsichtlich Größe und Frequenz bezogen. Ein "Atemschaltkreis" berechnet eine erste Stimulationsrate abhängig von der Impedanz. Weiterhin ist ein mechanischer Aktivitätssensor vorgesehen und ein Aktivitätskreis berechnet eine zweite Stimulationsrate. Eine Steuerschaltung berechnet aufgrund der ersten und zweiten Stimulationsrate und deren Kurzzeit- und Langzeitänderungen die Stimulationsrate mit der der Herzschrittmacher gesteuert wird.

Aus der US 5 360 436 ist eine Frequenzsteuerung eines Aktivitätsherzschrittmachers bekannt, bei der eine Vielzahl von Bewegungsmustern und Reaktionsmustern bei unterschiedlichen Belastungsstufen bei einer gesunden Person gemessen und in einem Herzschrittmacher gespeichert werden. Die Belastungszustände der Person mit Herzschrittmacher werden aber einen Aktivitätssensor, d.h. einen mechanischen nicht physiologischen Sensor erfasst und mit dem gespeicherten Muster verglichen, wobei die Pulsfrequenz des Musters, das am besten übereinstimmt zur Steuerung des Herzschrittmachers verwendet wird.

EP 0 452 733 A2 beschreibt einen Herzschrittmacher der zwei oder mehr Sensoren aufweist und abhängig von der Kombination der Sensorsignale gesteuert wird. Im Ausführungsbeispiel werden ein Aktivitätssensor mit schneller Änderung und ein physiologischer Sensor mit langsameren Änderungen z.B. ein Bluttemperatursensor oder Blutsauerstoffsensor verwendet.

Der Erfindung liegt die Aufgabe zugrunde, einen Herzschrittmacher zu schaffen, der ohne die Notwendigkeit funktionsspezifischer Signalanteile aus einem intrakardialen Messmischsignal herauszufiltrieren, genau und schnell den körperlichen Belastungszustand erfasst und entsprechend die Grundfrequenz der Stimulation steuert.

Diese Aufgabe wird erfindungsgemäß durch die kennzeichnenden Merkmale des Hauptanspruchs in Verbindung mit den Merkmalen des Oberbegriffs gelöst.

Der Herzschrittmacher gemäß der Erfindung umgeht das Problem der Filterung einzelner funktionsspezifischer Signalanteile aus einem Mischsignal, wie der intrakardialen Impedanz, indem auf eine exakte Diskriminierung verzichtet wird und statt dessen nur Folgen von bestimmten Signalparametern analysiert werden, die auf eine genau reproduzierbare Weise auf bestimmte Einflußgrößen, wie etwa die körperliche Belastung und die Pulsfrequenz des Herzens reagieren, so daß jede Wertefolge eine bestimmte belastungs- und frequenzabhängige Verlaufscharakteristik aufweist. Damit ergibt sich die Möglichkeit, bei Speicherung einer entsprechenden Zahl dieser Wertefolgen bei definierten Belastungen als Referenzwertefeld durch Vergleich einer oder mehrerer aktueller Wertefolgen mit den gespeicherten Werten den herrschenden Belastungsgrad zu detektieren. Dazu wird die gespeicherte Wertefolge festgestellt, die bezogen auf die jeweilige Grundfrequenz die größte Korrelation zur aktuellen Wertefolge hat. Diese Vorgehensweise benötigt dann keine genaue Analyse einzelner belastungstypischer Signalinhalte mehr.

In der bevorzugten Ausführungsform der Einrichtung wird jeder vorher ausgewählten Kombination aus definiertem Belastungszustand und Pulsfrequenz ein Referenzwertefeld zugeordnet, in das eine zugehörige Folge von Signalparametern physiologischer Meßsignale abgespeichert werden. Diese gespeicherten Werte werden dann als Bezugsgrößen (Muster-Schablone) benutzt, um durch den Vergleich mit aktuellen Meßsignalfolgen bzw. -mustern unterschiedliche Belastungssituationen zu detektieren. Das hier genutzte Verfahrensprinzip der Identifikation von Verlaufscharakteristiken bzw. Verlaufsmustern von physiologischen Meßwerten ist sinngemäß um so wirksamer, je störungsfreier die jeweiligen Wertefolgen reproduziert werden können und je charakteristischer ihr Verlauf ist. Die genau reproduzierbaren Wertemuster oder das Referenzwertefeld werden gewonnen, indem die jeweils eingestellte Stimulationsfrequenz moduliert wird und als Signalparameter nur die Signalanteile genutzt werden, die durch die Frequenzmodulation beeinflußt werden, so daß durch die Demodulation nichtmodulationssynchrone Störgrößen unterdrückt werden. Eine beispielhafte Form, eine Folge von Signalparametern zu gewinnen, besteht darin, unterschiedliche Arten und Grade der Frequenzmodulation durchzuführen, worauf bei anschließender Demodulation eine entsprechend große Zahl bzw. Folge von Werten gewonnen werden kann.

Durch die in den Unteransprüchen angegebenen Maßnahmen sind weitere Verbesserungen und Weiterbildungen möglich. Besonders vorteilhaft ist, daß eine schnelle und genaue Regelung der Stimulationsfrequenz an dem jeweiligen Belastungszustand vorgenommen werden kann.

Ein Ausführungsbeispiel der Erfindung ist in der Zeichnung dargestellt und wird in der nachfolgenden Beschreibung näher erläutert. Es zeigen:

Fig. 1          einen zeitlichen Verlauf der modulierten Stimulationsfrequenz bei unterschiedlichen Grundfrequenzen und unterschiedlichen Modulationsgraden bei Doppelpulsmodulation als Modulationsart,

Fig. 2          ein Referenzwertefeld als Belastungs-Stimulationsfrequenz-Koordinatenfeld mit verschiedenen Grundfrequenzen und unterschiedlichen Belastungsstufen,

Fign. 3 und 3a      die Darstellung eines Signalparameters nach Frequenzänderung entsprechend unterschiedlichen Modulationsgraden bzw. Abtastpunkten innerhalb des Pulsintervalls,

Fig. 4          ein Blockschaltbild einer Einrichtung nach einem Ausführungsbeispiel der vorliegenden Erfindung,

Fig. 5          ein Blockschaltbild einer Einrichtung nach einem weiteren Ausführungsbeispiel der vorliegenden Erfindung, und

Fig. 6          Kennlinien verschiedener intrakardialer Signale.

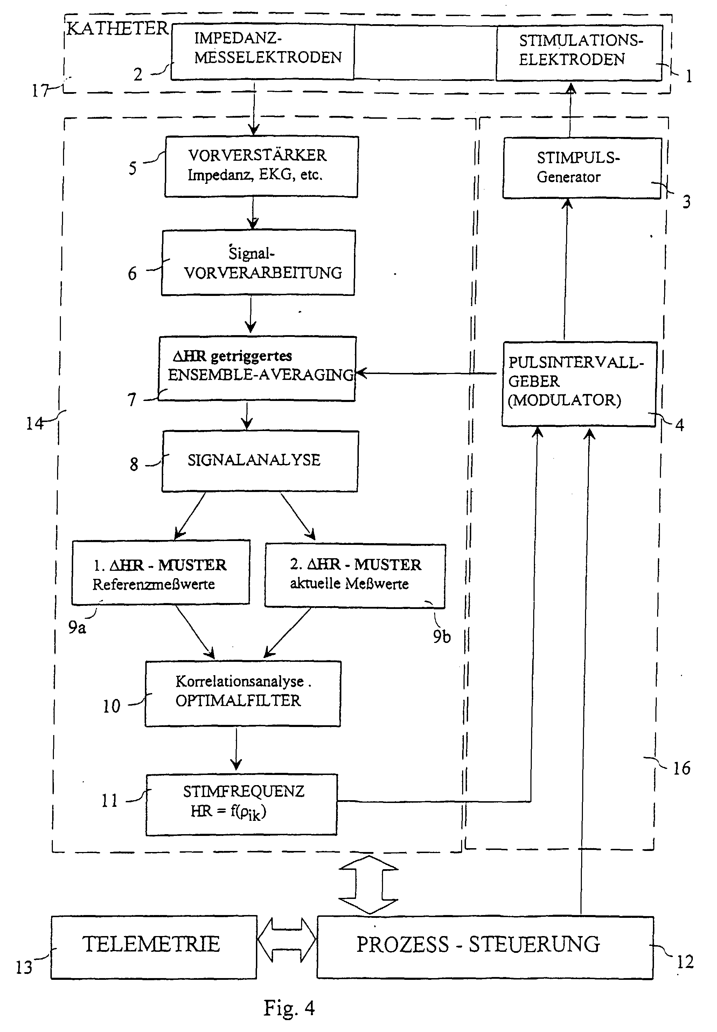

In Fig. 4 ist ein Blockschaltbild eines Herzschrittmachers dargestellt, der abhängig von Stimulationsimpulsen mit vorgebbarer Stimulationsfrequenz gesteuert wird. Der Herzschrittmacher enthält einen Stimulationsimpulsgenerator 3, der mit einer oder mehreren im Herzen des Patienten angeordneten Elektroden verbunden ist. Der Stimulationsimpulsgenerator 3 ist mit einer Regelstufe 4 verbunden, die die Größe und zeitliche Folge, das heißt die Frequenz der Stimulationsimpulse steuert. Die Stimulationselektroden 1 werden gleichzeitig als Meßelektroden für EKG- und Impedanzmessung genutzt, wobei zusätzliche Sensoren 2 (zum Beispiel zur Messung von Druck oder Sauerstoffgehalt) im Katheter integriert sein können. Die Elektroden bzw. Sensoren 1, 2 sind mit einer Verarbeitungs- und Auswerteeinheit 14 verbunden, in der die von den Elektroden 1 bzw. Sensoren 2 gelieferten Signale, wie später beschrieben wird, verarbeitet und ausgewertet werden. Die Verarbeitungs- und Auswerteeinheit 14 ist mit einer standardisierten Prozeßsteuerung 12 und einer Telemetrievorrichtung 13 verbunden, die Daten von der Verarbeitungseinheit 14 nach außen weiterleiten und umgekehrt. Der Herzschrittmacher wird über die Stimulationselektrode 1 mit einer durch die Regelstufe 4 vorgegebenen Stimulationsfrequenz gesteuert, wodurch Stimulationsimpulse an das Herz abgegeben werden. Diese Frequenz wird abhängig von unterschiedlichen Einstellparametern durch die Regelstufe eingestellt.

In Tabelle 1 sind die gebräuchlichsten Einstellparameter der Stimulationsfrequenz, die im Englischen mit heartrate ($HR_0$) bezeichnet wird und deren Änderungen ($\Delta HR_j$) aufgeführt, aus denen je nach Anwendungsfall bestimmte Kombinationsfolgen oder Muster zusammengestellt werden können. In der untersten Reihe der Tabelle 1 ist die Zahl einer normalen Kombination und in Klammern die der maximalen Kombinationsfälle angegeben.

Tabelle 1

| Einstellparameter der STIMULATIONS-FREQUENZ ($\mathbf{HR_o}$, $\mathbf{\Delta HR_j}$) | | | |
|---|---|---|---|
| ART der Stimulation (SM) | GRUNDWERT der Pulsfrequenz. ($HR_o$) | ART der Frequenzmodulation (FM) | GRAD der Frequenzmodulation. ($\Delta HR$) |
| PACING<br><br>1. Ventrikel<br><br>(2. Atrium)<br><br>(3. A / V)<br><br>4. SINUS | 1. 60- 80 P/min<br><br>2. 80 -100 P/min<br><br>3. 100-130 P/min<br><br>4. 130-160 P/min | 1.EINZELPULS positiv (+$\Delta HR$)<br><br>(2.EINZELPULS negativ (-$\Delta HR$))<br><br>3.DOPPELPULS (+/-$\Delta HR$) | 1. 5 %<br><br>2. 10 %<br><br>3. 20 %<br><br>(4. 40 %) |
| 2 (4) | 1-8 (1-16) | 2 (3) | 1-6 (1-12) |

Bei der Art der Stimulation SM ist prinzipiell zwischen Eigenrhythmus (SINUS) und künstlicher Stimulation, die wiederum in die Atrium(A)-, Ventrikel(V)-, und die kombinierte A-V-Stimulation getrennt wird, zu unterscheiden. Als Grundwerte (Grundfrequenz $HR_0$) der Stimulationsfrequenz werden zunächst nur vier Werte bestimmt, die jeweils aus einem Wertebereich gewählt wurden, der seinerseits einem der im folgenden definierten Belastungsbereiche zugeordnet wird. Bei Sinusrhythmus wird die jeweilige mittlere Pulsfrequenz von mehreren (> 4) aufeinanderfolgenden Pulsen als Bezugsgröße berechnet und einem der vier Frequenzbereiche zugeordnet.

Wie aus dem Stand der Technik bekannt ist, wird für eine Frequenzregelung der Stimulationsfrequenz letztere moduliert und es werden beispielsweise über die EKG-Messung die Reaktionen der intrakardialen Signale auf diese Modulation erfaßt und ausgewertet. Dabei kann die Art der Frequenzmodulation und der Grad der Frequenzmodulation oder beide geändert werden. Entsprechend Tabelle 1 wird bei den Modulationsarten beispielhaft nur zwischen typischen Formen der Einzelpulsmodulation unterschieden, bei der im periodischen Abstand von n Pulsen das Intervall zwischen zwei aufeinanderfolgenden Pulsen entweder verlängert (-$\Delta HR_j$) oder verkürzt (+$\Delta HR_j$) wird. Selbstverständlich können auch andere Arten der Frequenzmodulation in Betracht gezogen werden, der Einfachheit halber bezieht sich das Ausführungsbeispiel auf die Doppelpulsmodulation.

In der Tabelle 1 ist der Grad der Frequenzmodulation in Prozent angegeben, wobei die Bezugsgröße die Grundfrequenz $HR_0$ der Stimulationsimpulse ist. Es wurden für die graduell abgestufte Einstellung des Modulationsgrades Verkürzungen bzw. Verlängerungen des Pulsintervalls gewählt, die einer Frequenzdifferenz von ± 5, 10 und 20 % entsprechen.

In Tabelle 2 ist der Einstellparameter der körperlichen Belastung $P_k$ dargestellt. Es werden beispielhaft verschiedene Arten körperlicher Belastung angeführt, die im medizinischen Bereich für die Bewertung von Körperfunktionen als reproduzierbare Belastungszustände genutzt werden. Dementsprechend können derartige Belastungseinstellungen in Verbindung mit definierten Frequenzmodulationen Referenzwerte liefern, die zur allgemeinen Belastungsdetektion bei Herzschrittmachern benötigt werden. Der Grad der körperlichen Belastung ist eine individuell variable Größe, daher muß für die einzelne Person zunächst der maximale Leistungspegel ($N_{max}$) ermittelt werden, der als Bezugsgröße für zum Beispiel zwei weitere Belastungsstufen mit 1/3 und 2/3 $N_{max}$ dient.

Tabelle 2

| Einstellparameter der körperlichen BELASTUNG ($P_k$) | |
| --- | --- |
| ART der körp.Belastung | GRAD der körp.Belastung |
| 1. Sitzen (Fahrradergometer)<br>(2. Liegen (Fahrradergometer))<br>(3. Gehen (Laufband)) | 1. Ruhe<br>2. 1/3 $N_{max}$ (W)<br>3. 2/3 $N_{max}$ (W)<br>4. 1 $N_{max}$ (W) |
| 1 (3) | 1-4 (1-12) |

Grundlage der Idee der Erfindung liegt darin, daß für eine individuelle Person Meßwertfelder bei veränderlichen Belastungsgraden bzw. -stufen und bei unterschiedlichen Stimulationsfrequenzen gespeichert werden, die als Referenzwertefelder dienen und mit denen aktuelle Meßwerte verglichen werden, wobei das Ergebnis des Vergleichs für die Steuerung bzw. Regelung der Stimulationsfrequenz verwendet werden kann. Dabei kann die Art der den Meßwerten zugrundeliegenden intrakardialen Signale beliebig gewählt werden, es müssen nur jeweils dieselben Signalparameter bei den Referenzwertefeldern und den aktuellen Meßwerten verwendet werden.

Im folgenden wird die Art der Erstellung der Referenzwertefelder näher erläutert. In Tabelle 3 ist ein Belastungs-Stimulationsfrequenz (Grundfrequenz) -Koordinatenfeld (HR-P-Koordinatenfeld) dargestellt, wobei jedem Koordinantenpunkt, der durch eine Belastungssituation oder -stufe ($P_k$) und einen Grundwert der Stimulationsfrequenz ($HR_0$) bestimmt ist, eine Folge von Meßwerten $M_{ik}$ ($\Delta HR_j$) zugeordnet ist. Die Folge von Meßwerten, d.h. von intrakardialen Meßsignalen, wird abhängig von dem jeweiligen Frequenzmodulationsgrad ($\Delta HR_j$) bestimmt. Somit ergibt sich beispielhaft die in Tabelle 3 zusammengestellte Werteanordnung.

Tabelle 3

| PULSFREQUENZ BELASTUNG | $HR_1$ | $HR_2$ | $HR_3$ | .... | .... | $HR_{imax}$ |
| --- | --- | --- | --- | --- | --- | --- |
| $P_1$ | $M_{11}$ ($\Delta HR_j$) | $M_{12}$ ($\Delta HR_j$) | $M_{13}$ ($\Delta HR_j$) | | | $M_{1,imax}$ ($\Delta HR_j$) |
| $P_2$ | $M_{21}$ ($\Delta HR_j$) | $M_{22}$ ($\Delta HR_j$) | $M_{23}$ ($\Delta HR_j$) | | | $M_{2,imax}$ ($\Delta HR_j$) |
| ... | | | | | | |
| ... | | | | | | |
| $P_{kmax}$ | $M_{kmax,1}$ ($\Delta HR_j$) | $M_{kmax,2}$ ($\Delta HR_j$) | $M_{kmax,3}$ ($\Delta HR_j$) | | | $M_{kmax,imax}$ ($\Delta HR_j$) |

Für die Bestimmung des Referenzwertefeldes im vorliegenden Ausführungsbeispiel wird ein HR-P-Koordinatenfeld mit vier Grundwerten ($HR_0$) für die Stimulations-frequenz, die allgemein als Pulsfrequenz bezeichnet wird, und vier Werten ($P_k$) für die körperliche Belastungssituation bestimmt, wodurch sich 16 Ereignisfelder ergeben. Jedem dieser Felder wird eine Folge von Meßwerten $M_{ik}$ zugeordnet, die im vorliegenden Fall als Funktion verschiedener Stufen von Frequenzmodulationen ($\Delta HR_j$) berechnet werden.

Als Frequenzmodulation wird einerseits eine konstante Art der Modulation verwendet, bei der eine Kombination aus jeweils einer gleichgroßen positiven und negativen Frequenzänderung (+/- $\Delta$ HR) zweier aufeinanderfolgender Pulsintervalle (Doppelpulsmodulation) gewählt wird, und andererseits werden drei unterschiedliche Modulationsgrade von 5, 10 und 20 % verwendet. Jede Frequenzänderung wird nach einer bestimmten Anzahl von Pulsen wiederholt, um einen Mittelwert über eine bestimmte Pulszahl (m) bilden zu können, wobei bei dem Modulationsgrad von 5 % eine Anzahl $m_5$ = 16, bei dem Modulationsgrad von 10 % $m_{10}$ = 12 und bei dem Modulationsgrad von 20 % $m_{20}$ = 8 gewählt wird.

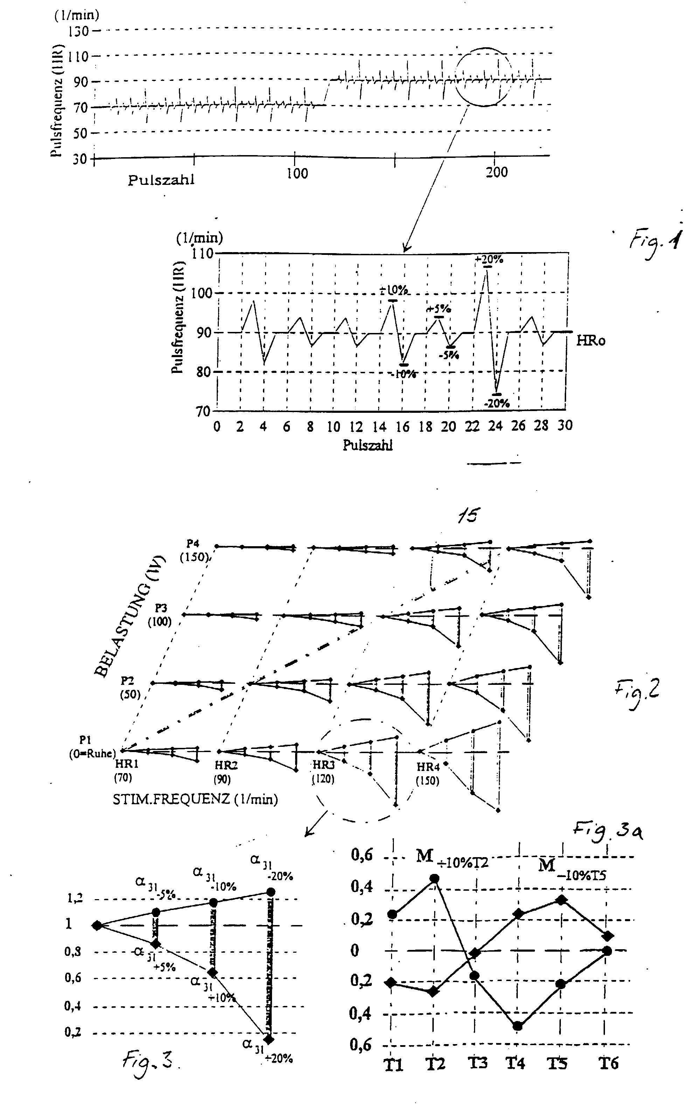

Werden zwischen den einzelnen Graden von Doppelpulsmodulationen zwei unmodulierte Pulsintervalle ($HR=HR_0$) verwendet, so ergibt sich der in Fig. 1 dargestellte zeitliche Verlauf der modulierten Stimulations- bzw. Pulsfrequenz bei zwei verschiedenen Grundwerten $HR_0$ (70 und 90 Pulse/min). Wie aus Fig. 1 zu erkennen ist, haben die niedriger modulierten Pulsintervalle eine höhere Wiederholfrequenz, weil Signaländerungen bei geringem Modulationsgrad sinngemäß einen niedrigeren Störabstand haben und dementsprechend über eine höhere Pulszahl integriert werden sollten. Außerdem sind beim gezeigten Beispiel Wiederholfrequenzen in einem bestimmten Bereich stochastisch variabel, um Interferenzen mit periodischen Einflußgrößen auf das intrakardiale Meßsignal, wie etwa die Atmung, zu verhindern.

In dem beschriebenen und dargestellten Ausführungsbeispiel wird als $\Delta HR_j$-abhängiger Signalparameter die Impedanz gewählt, und zwar der mittlere normierte Wert $\alpha_{ik}$ der maximalen Impedanzänderung $\Delta Z_{max}$, während eines Pulsintervalls (n+1) nach der Intervalländerung des Pulses (n). Der Mittelwert $\overline{\Delta Z}_{max}$ wurde dabei jeweils entsprechend der unterschiedlichen Wiederholfrequenzen über zum Beispiel $m_5=16$, $m_{10}=12$ und $m_{20}=8$ (m Anzahl der Pulse) berechnet. Damit ergibt sich für den jeweiligen Mittelwert:

$$\overline{\overline{\Delta Z}}_{max} \ (\Delta HR_j) \ = \ 1/m \ \sum_{n=1}^{m} \ \Delta Z_{max(n)}$$

mit

$$\Delta Z_{max(n)} = Z_{max(n)} - Z_{min(n)}.$$

Als normierter Wert $\alpha_{ik}$ wird z.B. der Quotient berechnet aus der Differenz zwischen dem $\overline{\Delta Z}_{max}$-Wert des jeweiligen Pulses (n+1), der durch die Frequenzänderung ($\Delta HR_j$) des vorhergehenden Pulsintervalls (n) beeinflußt wird und einem Bezugswert ($\Delta Z_{0max}$), der nicht durch eine Frequenzänderung beeinflußt wird zu $\Delta Z_{0max}$ entsprechend:

$$\alpha_{ik}(\Delta HR_j) = (\overline{\Delta Z}_{max} - \Delta Z_{Omax})/\Delta Z_{0max}.$$

In Fig. 2 ist ein der Tabelle 3 entsprechendes HR-P-Koordinatenfeld als Referenzwertefeld von charakteristischen Meßwertefolgen, die als normierte $\alpha_{ik}(\Delta HR_j)$-Folgen ausgebildet sind, dargestellt. Die Wertefolgen von 6 $\Delta HR$-abhängigen Signalparametern $\alpha_{ik}$ nach Frequenzänderungen mit einem Modulationsgrad $\Delta HR$ von +/- 5, 10, 20 %, wurden bei vier verschiedenen Grundfrequenzen (70, 90, 120, 150 Pulse/min) und vier Belastungsstufen (P1 - P4) entsprechend der Tabelle 2 bestimmt. Die Wertefolge $\alpha_{31}$ bei einer Grundfrequenz von 120 Pulse/min im Ruhezustand ist in Fig. 3 vergrößert dargestellt, wobei die Abweichungen bei den unterschiedlichen Modulationsgraden deutlich zu erkennen sind.

Ein Referenzwertefeld, wie es in Fig. 2 dargestellt ist, kann personenspezifisch gespeichert werden, wobei selbstverständlich zusätzlich oder an dessen Stelle weitere Felder mit unterschiedlichen Parametern sowohl hinsichtlich der Frequenzmodulation als auch der Belastung als auch des erfaßten Signalparameters gespeichert sein können.

Entsprechend dem Ausführungsbeispiel werden aus dem Referenzwertefeld nach Fig. 2 während der definierten Belastungstests, d.h. bei der Referenzmessung, die Wertemuster ausgewählt, die sich bei den als optimal bewerteten jeweiligen Stimulationsfrequenzen ergeben.

Eine physiologisch optimale Beziehung zwischen Belastung und Stimulationsfrequenz wird etwa durch die Gleichung

$$HRo = HR_{min} + c \, P_k$$

beschrieben, wobei HRo die Grundfrequenz und $HR_{min}$ und c individuell einstellbare Konstanten sind. Daraus ergibt sich für jede Grundfrequenz HRo ein als optimal definiertes Änderungsmuster entsprechend der in Fig. 2 gestrichelt gezeichneten Kennlinie 15, d.h. bei vier Grundfrequenzen vier Referenzwertemuster. Diese Referenzwertemuster werden personenspezifisch vorgegeben. Falls es sich für den Patienten als vorteilhaft erweist, können auch andere Referenzwertemuster aus dem gesamten Referenzwertefeld ausgewählt werden, die seinem körperlichen Zustand angemessen sind.

Zur Bestimmung der aktuellen nicht bekannten körperlichen Belastungsstufe der den Herzschrittmacher tragenden Person wird wie bei der Referenzmessung eine normierte Meßwertfolge $\alpha_{ik}$ bei einer vorgegebenen Grundfrequenz und bei einer Art der Frequenzmodulation und den verschiedenen Modulationsgraden durch Messung und Berechnung bestimmt. Zur Bestimmung der körperlichen Belastungsstufen mit Hilfe der aktuell gemessenen $\Delta HR$-bezogenen Wertefolge eignet sich grundsätzlich jedes Identifikationsverfahren, wobei im Ausführungsbeispiel die sogenannte Optimalwert-Filterung beschrieben wird. Dabei wird das Quadrat der mittleren Differenz $(\Delta \alpha_{ik})^2$ zwischen den Werten der aktuellen Meßfolge und denen der Referenzwertfolgen bestimmt und die Summe $\rho_{ik}$ aller so ermittelten

Werte gebildet:

$$\rho_{ik} = \sum_{a=1}^{6} (\Delta\alpha_{ik})_{a}^{2} = \sum (\alpha_{ik} - \alpha_{ikref})_{a}^{2}$$

Allgemein gilt, daß der Koordinatenpunkt, für den $\rho_{ik}$ am kleinsten wird, die aktuelle Meßwertfolge definiert, die am besten mit der Referenzwertefolge korreliert und damit den jeweiligen Belastungsgrad kennzeichnet, d.h.:

$$P_k = P_k (\rho_{ik} \rightarrow min).$$

Falls $\rho_{ik}$ über einen bestimmten Schwellenwert liegt, wird die Regelung durchgeführt und entsprechend dem Wert $\rho_{ik}$ die Grundfrequenz erhöht bzw. erniedrigt und $\alpha_{ik}$ gemessen und mit dem nächst höheren bzw. niedrigeren Referenzwertemuster verglichen. Dadurch wird entsprechend den gespeicherten Mustern die optimale Grundfrequenz für die aktuelle Belastung eingestellt.

Die den oben beschriebenen Verarbeitungsvorgang durchführende Einrichtung ist in Fig. 4 dargestellt, wobei die Regelstufe 4 den Stimulationsimpulsgenerator hinsichtlich der Größe und zeitlichen Folge (Frequenz der Stimulationsimpulse) steuert, die an die Stimulationselektroden weitergegeben werden.

Die Vorrichtung zur Verarbeitung und Auswertung 14 umfaßt alle zur Verarbeitung der über die Meß- bzw. Elektrodenanordnung 1, 2 abgeleiteten Meßsignale erforderlichen Funktionsstufen. Dazu zählen im wesentlichen ein Vorverstärker 5 mit A/D-Wandler, eine Signalvorverarbeitung 6, die nicht Pulsintervall- (d.h. Stimulationsfrequenz-) bezogen ist, und eine Pulsintervall-getriggerte Signalverarbeitung 7. Bei Änderung der Pulsintervalldauer gibt die Frequenzregelstufe 4 einen Triggerimpuls an die Verarbeitungsstufe 7, die dann eine Mittelwertbildung durchführt. Eine folgende Signalanalysestufe 8 erfaßt die Pulsintervall-bezogenen Werte der gemittelten Signalverläufe, die für die jeweils gewünschte Analyse am charakteristischsten sind, wie etwa die maximale Amplitudenänderung der intrakardialen Impedanz pro Pulsintervall. Der Signalanalysestufe 8 ist eine Speicherstufe 9 nachgeschaltet, wobei in dem Speicherbereich 9a das Referenzwertefeld bzw. die Referenzwertefelder abgelegt sind und in dem Speicherbereich 9b die aktuellen Meßwertefolgen zwischengespeichert werden. Eine Vergleichsstufe 10 analysiert die Korrelation $\rho_{ik}$ zwischen den Referenzwertefeldern und den aktuellen Meßwertfolgen und gibt das Ergebnis an die Frequenzregelstufe 11 weiter. Der von der Frequenzregelstufe als Funktion des Korrelationsfaktors $\rho_{ik}$ bestimmte Grundwert $HR_0$ der Stimulationsfrequenz steuert den Pulsintervallgeber 4 in Kombination mit der Prozeßsteuerung 12, die ihrerseits nach über die Telemetrie 13 vorgebbarem Programm die Dauer, Art und den Grad der Frequenzmodulation bestimmt.

In dem oben beschriebenen Ausführungsbeispiel wurden die Meßwertfolgen mit der in Fig. 2 dargestellten Verlaufsform auf der Grundlage der Impedanzmessung bestimmt. Es können jedoch auch andere Meßparameter bzw. kombinierte Parameter gewählt werden, wobei dann andere Verlaufsformen einem Referenzwertefeld entsprechend Fig. 2 zugrundeliegen.

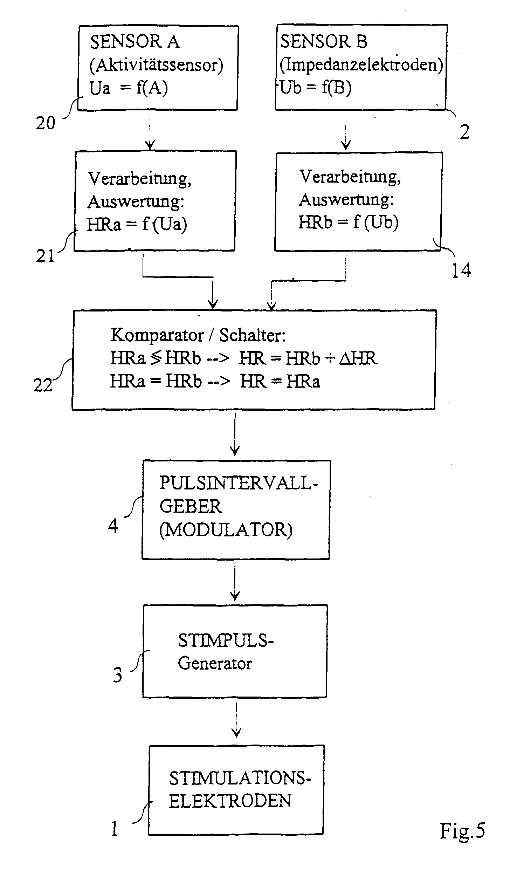

Die Anpassung des Herzschrittmachers an geänderte Belastungszustände ist relativ langsam, da die Reaktionen auf die Modulation abgewartet werden müssen und der Meßzyklus relativ lang ist. Dies kann durch eine Kombination mit einem bewegungsabhängigen Sensor entsprechend Fig. 5 verbessert werden.

Bewegungsabhängige Sensoren werden in Aktivitätsschrittmachern eingesetzt, die jedoch den funktionellen Nachteil haben, daß sie im Prinzip auch durch nichtphysiologische Größen (Beschleunigung, Schwingungen) beeinflußt werden und unspezifisch reagieren. Die Belastungsdetektion nach Fig. 5 ermöglicht eine Korrektur nicht physiologischer Änderungen der Stimulationsfrequenz.

In Fig. 5 ist mit 20 ein beispielsweise als Piezoelement ausgebildeter Bewegungssensor bezeichnet, der mit einer Signalauswertestufe 21 verbunden ist, deren Ausgang an einen Komparator/Schalter 22 angeschlossen ist, der mit dem Pulsintervallgeber bzw. Modulator 4 verbunden ist, der den Stimulationsimpulsgenerator 3 steuert. Die Stimulationspulse werden den Stimulationselektroden 1 zugeführt.

Parallel dazu wird die oben beschriebene Anordnung wirksam, d.h. über die Impedanzmeßelektroden 2 wird die Impedanz gemessen und der Signalverarbeitungsstufe 14 zugeführt, die eine Verarbeitung der erfaßten Meßparameter, hier der Impedanz im Vergleich mit den oben beschriebenen gespeicherten Referenzwerten durchführt und die geeignete Stimulationsfrequenz $HR_b$ als Funktion der aktuellen Impedanzwerte berechnet.

Die Funktionsweise der Vorrichtung nach Fig. 5 ist wie folgt.

Im Normalfall wird über die Impedanzelektroden 2 und die Signalverarbeitungsstufe 14 laufend die Grundfrequenz $HR_b$ ohne die Durchführung der Modulation erfaßt und entsprechend der Stimulationsimpulsgenerator 3 gesteuert.

Wenn der Bewegungssensor 20 eine geänderte Bewegung erfaßt, wird in der Signalauswertestufe 21 festgestellt, ob die Änderung über einen vorgegebenen Schwellenwert liegt und entsprechend dem bekannten Stand der Technik wird zu der Bewegung eine Grundfrequenz $HR_a$ festgelegt. In dem Komparator/Schalter 22 wird bestimmt, ob diese Grundfrequenz größer oder kleiner als die Grundfrequenz $HR_b$ ist, die laufend über die Impedanzmeßvorrichtung erfaßt wird.

Wenn dies der Fall ist, wird an den Pulsintervallgeber 4 ein Signal gegeben, eine Modulation $HR = HR_b + \Delta HR$ durchzuführen, wobei der Stimulationspulsgenerator 3 die entsprechenden Impulse abgibt, und es wird eine Regelung nach der in Zusammenhang mit den Fign. 1 bis 4 dargelegten Beschreibung durchgeführt. Im Komparator 22 wird festgestellt, ob die über die Signalverarbeitungsstufe 14 bestimmte Grundfrequenz $HR_b$ mit der abhängig vom Bewegungssensor 20 bestimmten Frequenz $HR_a$ übereinstimmt. War die Bewegungsänderung durch nichtphysiologische Größen bewirkt worden, so ändert der Pulsintervallgeber 4 die Grundfrequenz auf den über die Impedanz eingestellten Wert $HR_b$. Anderenfalls wird die Grundfrequenz $HR_a$ vorgegeben und die Modulation beendet.

In dem Beispiel nach Fig. 2 und 3 wird das Referenzwertefeld durch Impedanzmeßwerte gebildet, die unter Verwendung einer Modulationsart bei unterschiedlichen Grundfrequenzen und Modulationsgraden sowie unterschiedlichen Belastungsstufen erfaßt wurden, wodurch ein "zweidimensionales" Meßwertefeld gebildet wird. Es ist auch möglich, ein "dreidimensionales" Meßwertefeld zu wählen, indem beispielsweise als zusätzlicher Parameter eine veränderliche Modulationsart verwendet wird.

Auch kann in einem weiteren Ausführungsbeispiel anstelle der veränderlichen Modulationsgrade unterschiedliche Abtastzeitpunkte innerhalb des Pulsintervalls erfaßt werden. Die Genauigkeit der Korrelationsanalyse verbessert sich mit der Zahl der erfaßten charakteristischen Meßwerte. Dies bedeutet, daß zur Erstellung der Meßwertefolge entweder die Zahl der Modulationsgrade erhöht werden muß oder die pro Modulationsgrad erfaßten Meßwerte. Da es physiologischer ist, die Zahl und Größe der genutzten Modulationsgrade möglichst klein zu halten, um den natürlichen Modulationsbereich der Pulsfrequenz so wenig wie möglich zu überschreiten, sollte die Tatsache genutzt werden, daß die Reizausbreitung sowie Druck-Volumen-Funktion des Herzens vier charakteristische Phasen durchläuft, die jeweils spezifisch durch die Frequenzmodulation beeinflußt werden. Dementsprechend ist es zweckmäßig, bei intrakardialen Meßwerten, die wie die Impedanz vom Druck- bzw. Volumenverlauf abhängen, pro erfaßtem Pulsintervall mindestens zwei Meßwerte, zum Beispiel Maximum während der Systole und Minimum während der Diastole, zu messen.

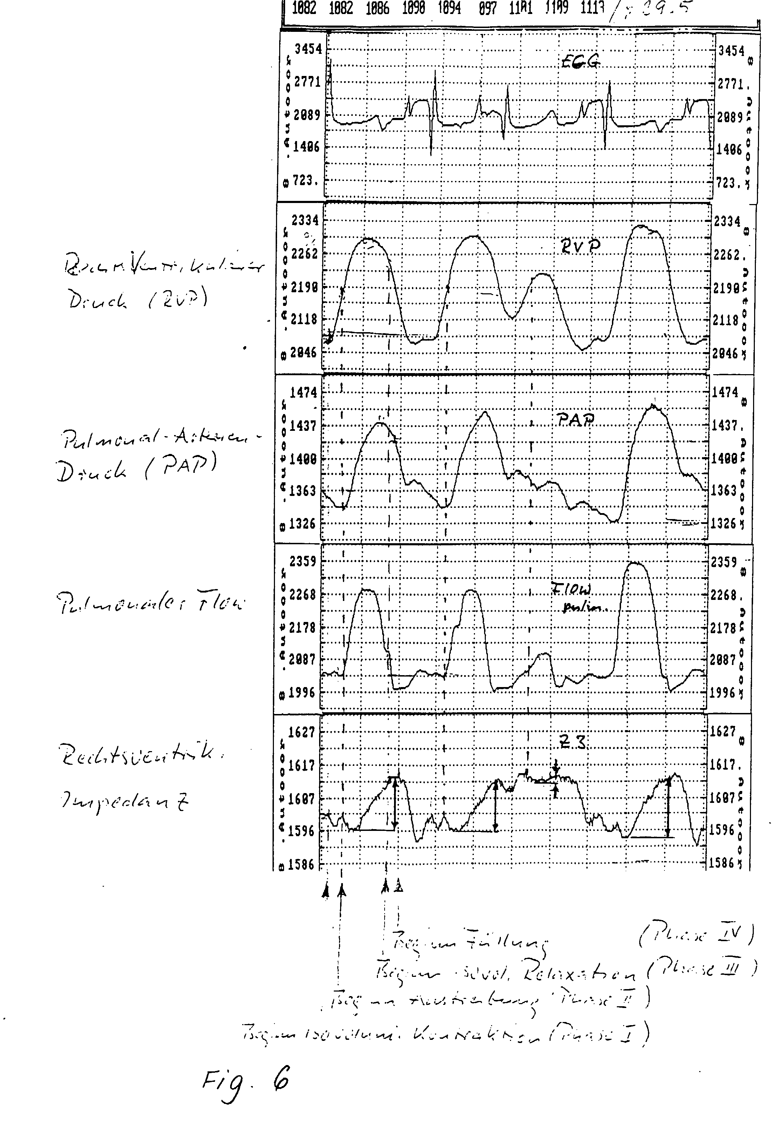

In Fig. 6 sind Kennlinien von unterschiedlichen intrakardialen Signalen über das Pulsintervall bzw. die Herzperiode dargestellt und die vier Phasengrenzen der Anspannungszeit (isovolumentrische Kontraktion), der Austreibungszeit, der Entspannungszeit (isovolumentrische Relaxation) und der Füllungszeit sind an der Abszisse bezeichnet. Die Kennlinien zeigen von oben nach unten unterschiedliche Arten von intrakardialen Signalen, nämlich das EKG (EEG), den rechtsventrikulären Druck (RvP), den Pulmonal-Arterien-Druck (PAP), den Pulmonalen Flow (Flow pulm) und die rechtsventrikuläre Impedanz.

Als Abtastzeitpunkt können Zeitpunkte während der Herzperiode bzw. dem Pulsintervall gewählt werden, bei denen die einzelnen Arten von Signalen abhängig von der Modulationsart und dem Modulationsgrad einen kennzeichnungskräftigen Verlauf aufweisen. Die Abtastzeitpunkt können für die unterschiedlichen Arten von Signalen unterschiedlich sein.

In der Praxis ist es vorteilhafter, den Aufwand für eine pulsphasen-spezifische Abtastung zu umgehen und statt dessen das Meßsignal an fest definierten Zeitpunkten während des Pulsintervalls abzutasten.

Wenn beispielsweise die Frequenzmodulation mit nur einem Modulationsgrad von zum Beispiel +/-10 % durchgeführt wird, dafür aber zum Beispiel sechs Abtastpunkte innerhalb des Pulsintervalls genutzt werden, ergibt sich analog zu Fig. 3 der Meßwertefolge nach Fig. 3a.

## Patentansprüche

1. Herzschrittmacher mit Stimulationselektroden (1) zum Stimulieren des Herzens mit Impulsen einer variablen Grundfrequenz, mit einer Sensorvorrichtung (1, 2) zum Erfassen von die Herzfunktion betreffenden Messwerten intrakardialer Signale und mit einer Vorrichtung (14) zur Verarbeitung und Auswertung der Messwerte, die einen Speicher zum Speichern von vorgegebenen, körperliche Belastungen berücksichtigende Referenzwerte aufweist, wobei die Grundfrequenz unter Verwendung der Referenzwerte veränderbar ist, **dadurch gekennzeichnet,** daß eine Vorrichtung (4) zur Modulation der Grundfrequenz mit mindestens einer Modulationsart und mindestens einem Modulationsgrad vorhanden ist, daß die Sensorvorrichtung (1, 2) bei unterschiedlichen vorgebbaren Stufen $(P_k)$ körperlicher Belastung bei unterschiedlichen vorgebbaren Grundwerten $(HR_0)$ der Stimulationsfrequenz und mindestens einer Modulationsart der Stimulationsfrequenz sowie unterschiedlichen vorgebbaren Modulationsgraden oder bei mindestens einer Modulationsart und mindestens einem Modulationsgrad der Stimulationsfrequenz sowie unterschiedlichen Abtastpunkten innerhalb des Pulsintervalls jeweils mindestens einen Messwert intrakardialer Signale erfasst und die dadurch entstehenden Messwertfolgen als Referenzwertefeld ($\alpha_{ikref}$) in dem Speicher gespeichert werden, daß die Sensorvorrichtung (1, 2) aktuelle intrakardiale Signale bei einer der Grundfrequenzen der Stimulationspulse sowie der Modulationsart und den vorgegebenen unterschiedlichen Modulationsgraden der Stimulationsfrequenz bzw. Abtastpunkten innerhalb des Pulsintervalls als Messwertfolge ($\alpha$ik) mißt und daß die Vorrichtung (14) zur Verarbeitung und Auswertung eine Korrelation der aktuellen Messwertfolge mit den als Referenzwertefeld gespeicherten Messwertfolgen vornimmt, wobei die Belastungsstufe derjenigen Messwertfolge des Referenzwertefeldes als aktuelle Belastungsstufe bestimmt wird, die die größte Korrelation zu der aktuellen Messwertfolge aufweist und daß die Grundfrequenz entsprechend geändert wird.

2. Herzschrittmacher nach Anspruch 1, dadurch gekennzeichnet, daß die Einstellung der Grundfrequenz der Stimulationspulse entsprechend einer vorgegebenen Kennlinie (15) der Grundfrequenz in Abhängigkeit von der Belastung bzw. entsprechend vorgegebener personenspezifischer Referenzwertemuster vorgenommen wird.

3. Herzschrittmacher nach Anspruch 1 oder 2, dadurch gekennzeichnet, daß die Stimulationsfrequenz derart moduliert wird, daß das Zeitintervall ($\Delta t_M$) zwischen nur zwei aufeinanderfolgenden Stimulationsimpulsen gegenüber einem als Grundwert definierten mittleren Intervall verkürzt oder verlängert wird und daß dieser Vorgang periodisch nach einer bestimmten Zahl (m) von Impulsen wiederholt wird.

4. Herzschrittmacher nach einem der Ansprüche 1 bis 3, dadurch gekennzeichnet, daß als intrakardiales Messsignal die Spannungen gewählt werden, die ohne (EKG) und/oder mit Stromzufuhr (Impedanz) über die Elektroden ein- oder mehrpoliger Stimulationskatheter ableitbar sind.

5. Herzschrittmacher nach einem der Ansprüche 1 bis 4, dadurch gekennzeichnet, daß die Extremwerte des intrakardialen Signals oder dessen erste Ableitung innerhalb eines systolischen oder diastolischen Zeitintervalls oder aber die Zeitabstände zwischen dem Beginn des Stimulationspulses und dem Erreichen eines der Extremwerte gewählt werden.

6. Herzschrittmacher nach Ansprüche 1 bis 5, dadurch gekennzeichnet, daß als frequenzmodulationsabhängige Messgröße die Änderungen des jeweils genutzten intrakardialen Messsignals gewertet werden, die sich aus der Analyse des Signalverlaufs während der der Intervalländerung folgenden zwei Pulsintervalle ergeben, wobei entweder die Differenz zwischen dem Grundwert und dem durch die Modulation beeinflußten Wert während des einen und/oder des anderen Pulsintervalls oder der in Bezug auf den Grundwert normierte Änderungswert ermittelt wird.

7. Herzschrittmacher nach einem der Ansprüche 1 bis 6, dadurch gekennzeichnet, daß der Mittelwert des jeweils genutzten Änderungswertes über mehrere Änderungsperioden bestimmt wird.

8. Herzschrittmacher nach einem der Ansprüche 1 bis 7, dadurch gekennzeichnet, daß die Modulation der Stimulationsfrequenz mit der Verarbeitung und Auswertung der Messsignale in bestimmten Zeitintervallen wirksam wird, die abhängig von der Auswertung eines oder mehrerer zusätzlicher, im Herzschrittmacher erfassbarer Messparameter gestartet werden.

9. Herzschrittmacher nach Anspruch 8, dadurch gekennzeichnet, daß der zusätzliche Messparameter nicht direkt mit der Herz-Kreislauffunktion des Körpers gekoppelt ist und unphysiologisch reagiert.

10. Herzschrittmacher nach Anspruch 9, dadurch gekennzeichnet, daß die Körperbewegung von einem Bewegungssensor erfasst wird.

11. Herzschrittmacher nach einem der Ansprüche 1 bis 10, dadurch gekennzeichnet, daß bei Erfassung des zusätzlichen Messparameters die Modulation und die Auswertung mit Hilfe der von der Frequenzmodulation abhängigen Referenzmuster durchgeführt wird, und ein Vergleich der so gefundenen Grundfrequenz mit einer Grundfrequenz, die aufgrund der Auswertung des zusätzlichen Messparameters bestimmt wird, durchgeführt wird.

## Claims

1. Cardiac pacemaker with stimulation electrodes (1) for the stimulation of the heart with impulses of a variable base frequency, having a sensor device (1, 2) to read intracardiac signals relating to heart function, and having a device (14) to process and evaluate the readings which has a memory for the storage of preset reference values which take into account levels of physical effort; the base frequency can be modified on the basis of the reference values; in which

there is a device (4) to modulate the base frequency, having at least one modulation mode and at least one modulation level;

the sensor device (1, 2) takes at least one reading of intracardiac signals in each case at varying presettable levels ($P_k$) of physical effort at varying presettable basic values ($HR_0$) of the stimulation frequency and at at least one modulation mode of the stimulation frequency, and at varying presettable modulation levels or at at least one modulation mode and at least one modulation level of the stimulation frequency and at varying scanning points within the pulse interval, the series of measurement readings thus created being stored as a reference value field ($\alpha_{ikref}$) in the memory;

the sensor device (1, 2) measures current intracardiac signals at one of the base frequencies of the stimulation pulses and at the modulation mode and the preset varying modulation levels of the stimulation frequency and/ or scanning points within the pulse interval as a series of measurement readings ($\alpha ik$);

the device (14) for processing and evaluation correlates the current measurement series with the series stored as a reference value field, whereby the current effort level is defined as being the effort level of the reference value field measurement series which has the closest correlation with the current measurement series

and the base frequency is modified accordingly.

2. Cardiac pacemaker as in Claim 1, in which the base frequency of the stimulation pulses is set in accordance with a predetermined characteristic curve (15) of the base frequency, depending on the effort and/or in accordance with predetermined patient-specific reference value patterns.

3. Cardiac pacemaker as in Claims 1 or 2, in which the stimulation frequency is modulated in such a way that the time interval ($\Delta t_M$) between only two consecutive stimulation impulses is extended or reduced in relation to a defined average interval which serves as a base value, and in which this process is repeated periodically after a specified number (m) of impulses.

4. Cardiac pacemaker as in one of Claims 1 to 3, in which the voltages selected as intracardiac measuring signals are derivable - without (ECG) and/or with electric current supply (impedance) - by means of the electrodes of a single- or multipolar stimulation catheter.

5. Cardiac pacemaker as in one of Claims 1 to 4, in which the peak values of the intracardiac signal or its first derivation are selected within a systolic or diastolic time interval, or the time intervals are selected between the beginning of the stimulation pulse and the achievement of one of the peak values.

6. Cardiac pacemaker as in Claims 1 to 5, in which the modifications - which are measured variables dependent on frequency modulation - to the intracardiac measuring signal being used in each case are evaluated, these modifications arising from the analysis of the signal progression during the two pulse intervals following the interval change, during which process either the difference between the base value and the value affected by the modulation during one and/or the other pulse interval, or the modified value normalized in relation to the base value, are established.

7. Cardiac pacemaker as in one of Claims 1 to 6, in which the average value of the modified value used in each case is established over several modification periods.

8. Cardiac pacemaker as in one of Claims 1 to 7, in which the modulation of the stimulation frequency with the processing and evaluation of the measuring signals takes effect at specific time intervals, which are instigated in dependence on the evaluation of one or more additional measurement parameters which are detectable in the pacemaker.

9. Cardiac pacemaker as in Claim 8, in which the additional measurement parameter is not directly linked to the cardiac circulatory function of the body and reacts in a non-physiological manner.

10. Cardiac pacemaker as in Claim 9, in which body movement is detected by a motion sensor.

11. Cardiac pacemaker as in one of Claims 1 to 10, in which - when detecting the additional measurement parameter - modulation and evaluation is carried out with the aid of the reference pattern which is dependent on the frequency modulation, and a comparison is carried out between the base frequency thus established and a base frequency determined on the basis of the evaluation of the additional measurement parameter.

**Revendications**

1. Stimulateur cardiaque comportant des électrodes de stimulation (1) servant à stimuler le coeur avec des impulsions ayant une fréquence de base variable, et comportant un dispositif de détection (1,2) servant à détecter des valeurs de mesure, qui concernent le fonctionnement du coeur, de signaux intracardiaques et un dispositif (14) pour traiter et exploiter les valeurs de mesure, qui comporte une mémoire pour mémoriser des valeurs de référence prédéterminées qui prennent en compte des contraintes corporelles, la fréquence de base pouvant être modifiée moyennant l'utilisation des valeurs de référence, caractérisé en ce qu'il est prévu un dispositif (4) servant à moduler la fréquence de base avec au moins un type de modulation et au moins un degré de modulation, pour différents échelons pouvant être prédéterminés ($P_k$) de contrainte corporelle et pour différentes valeurs de base pouvant être prédéterminées ($HR_0$) de la fréquence de stimulation et d'au moins un type de modulation de la fréquence de modulation ainsi que pour différents degrés de modulation pouvant être prédéterminés ou dans le cas d'au moins un mode de modulation et d'au moins un degré de modulation de la fréquence de stimulation ainsi qu'à des instants différents d'échantillonnage pendant l'intervalle des impulsions, le dispositif de détection (1,2) enregistre respectivement au moins une valeur de mesure de signaux intracardiaques et que les suites de valeurs de mesure, qui sont obtenues de ce fait, sont mémorisées en tant que champ de valeurs de référence ($\alpha_{ikref}$) dans la mémoire, que le dispositif de détection (1,2) mesure des signaux intracardiaques réels pour l'une des fréquences de base des impulsions de stimulation ainsi que pour le type de modulation et les différents degrés prédéterminés de modulation de la fréquence de stimulation ou des points d'échantillonnage pendant l'intervalle des impulsions en tant que suite de valeurs de mesure ($\alpha ik$) et que le dispositif de traitement et d'exploitation (14) réalise une corrélation de la suite réelle de valeurs de mesure avec les suites de valeurs de mesure mémorisées en tant que champ de valeurs de référence, l'échelon de contrainte de la suite de valeurs de mesure du champ de valeurs de référence, qui présente la corrélation maximale avec la suite actuelle de valeurs de mesure, étant déterminé en tant qu'échelon actuel de contrainte, et la fréquence de base étant modifiée de façon correspondante.

2. Stimulateur cardiaque selon la revendication 1, caractérisé en ce que le réglage de la fréquence de base de l'impulsion de stimulation est réalisée conformément à une courbe caractéristique prédéterminée (15) de la fréquence de base en fonction de la contrainte ou en fonction d'un modèle prédéterminé de valeurs caractéristiques, qui est spécifique à la personne.

3. Stimulateur cardiaque selon la revendication 1 ou 2, caractérisé en ce que la fréquence de stimulation est modulée de telle sorte que l'intervalle de temps ($\Delta t_M$) entre seulement deux impulsions de stimulation successives est raccourci ou prolongé par rapport à un intervalle moyen défini en tant que valeur de base et que ce processus est répété périodiquement au bout d'un nombre déterminé (m) d'impulsions.

4. Stimulateur cardiaque selon l'une des revendications 1 à 3, caractérisé en ce qu'on choisit comme signal de mesure intracardiaque les tensions qui peuvent être obtenues sans apport de courant (électrocardiogramme) et/ou avec apport de courant (impédance) par l'intermédiaire des électrodes de cathéters de stimulation unipolaires ou multipolaires.

5. Stimulateur cardiaque selon l'une des revendications 1 à 4, caractérisé en ce que les valeurs extrêmes du signal intracardiaque ou la dérivée première de ce signal sont choisies au cours d'un intervalle de temps systolique ou diastolique, ou bien que les intervalles de temps sont choisis entre le début de l'impulsion de stimulation et le moment où l'une des valeurs extrêmes est atteinte.

6. Stimulateur cardiaque selon les revendications 1 à 5, caractérisé en ce que les variations du signal de mesure intracardiaque respectivement utilisées, qui sont obtenues à partir de l'analyse de l'allure du signal pendant la variation de l'intervalle de deux intervalles successifs des impulsions, sont évaluées en tant que grandeurs de mesure qui dépend de la modulation de fréquence, la différence entre la valeur de base et la valeur influencée par la modulation pendant un intervalle et/ou l'autre intervalle des impulsions ou la valeur de modification normalisée d'une manière rapportée à la valeur de base étant déterminée.

7.  Stimulateur cardiaque selon l'une des revendications 1 à 6, caractérisé en ce que la valeur moyenne de la valeur de modification respectivement utilisée est déterminée sur plusieurs périodes de variation.

8.  Stimulateur cardiaque selon l'une des revendications 1 à 7, caractérisé en ce que la modulation de la fréquence de stimulation devient active avec le traitement et l'évaluation des signaux de mesure dans des intervalles de temps déterminés, qui démarrent en fonction de l'évaluation d'un ou de plusieurs paramètres supplémentaires de mesure pouvant être enregistrés dans le stimulateur cardiaque.

9.  Stimulateur cardiaque selon la revendication 8, caractérisé en ce que le paramètre de mesure supplémentaire n'est pas couplé directement à la fonction du circuit cardiaque du corps et réagit d'une manière non physiologique.

10. Stimulateur cardiaque selon la revendication 9, caractérisé en ce que le déplacement du corps est enregistré par un capteur de déplacement.

11. Stimulateur cardiaque selon l'une des revendications 1 à 10, caractérisé en ce que lors de l'enregistrement du paramètre de mesure supplémentaire, l'évaluation et l'exploitation à l'aide du modèle de référence qui dépend de la modulation de fréquence, sont exécutées, et une comparaison de la fréquence de base ainsi trouvée à une fréquence de base, qui est déterminée sur la base de l'exploitation du paramètre de mesure supplémentaire, est effectuée.

Fig. 1

Fig. 2

Fig. 3a

Fig. 3

Fig. 4

| SENSOR A | SENSOR B |
|---|---|
| (Aktivitätssensor) | (Impedanzelektroden) |
| $Ua = f(A)$ | $Ub = f(B)$ |

20

2

| Verarbeitung, | Verarbeitung, |
|---|---|
| Auswertung: | Auswertung: |
| $HRa = f(Ua)$ | $HRb = f(Ub)$ |

21

14

Komparator / Schalter:

$HRa \lesseqgtr HRb \dashrightarrow HR = HRb + \Delta HR$

$HRa = HRb \dashrightarrow HR = HRa$

22

PULSINTERVALL-
GEBER
(MODULATOR)

4

STIMPULS-
Generator

3

STIMULATIONS-
ELEKTRODEN

1

Fig.5

15

Fig. 6